# FASCICULE DE BREVET EUROPEEN

(11) **EP 0 795 321 B1**
(45) Date de publication et mention de la délivrance du brevet: **03.05.2000**
(21) Numéro de dépôt: 97400226.3
(22) Date de dépôt: 31.01.1997
(51) Int. Cl.: A61K 7/48, C08L 5/14, A61K 47/36, A61K 7/06

(54) **Utilisation d'alkyléthers de polysaccharides pour stabiliser des émulsions eau-dans-huile exemptes d'électrolytes**
Verwendung von Polysaccharidalkylethern zur Stabilisierung von elektrolytfreien Wasser-in-Öl Emulsionen
Use of polysaccharide alkyl ethers for stabilising water-in-oil emulsions in the absence of electrolytes

(30) Priorité: 12.03.1996 FR 9603095
(43) Date de publication de la demande: 17.09.1997
(73) Titulaire: L'OREAL, 75008 Paris (FR)
(72) Inventeur: Simon, Pascal, 94400 Vitry-sur-Seine (FR); Gagnebien, Didier, Westfield 07090, New-Jersey (US)
(74) Mandataire: Lhoste, Catherine

(56) Documents cités:
- EP-A- 0 281 360
- EP-A- 0 682 936
- EP-A- 0 708 114
- RESEARCH DISCLOSURE, no. 37807, Octobre 1995, page 642 XP002018143 MAJEWICZ ET AL.: "oil-based cosmetic and therapeutic compositions containing ethylguar"
- RESEARCH DISCLOSURE, no. 38413, 10 Avril 1996, pages 235-236, XP002033228 MAJEWICZ ET AL.: "ethyl galactomannan fim properties for use in personal care applications"
- GENNARO AR ET AL., 'REMINGTON: THE SCIENCE AND PRACTICE OF PHARMACY', 19. ed. 1995, pages 289-290, MACK PUBLISHING COMPANY, EASTON, PENNSYLVANIA

## Description

La présente invention se rapporte à une émulsion eau-dans-huile fluide stable, et à son utilisation dans les domaines cosmétique, dermatologique, vétérinaire et/ou agro-alimentaire. Elle peut se présenter notamment sous forme d'une crème blanche ou colorée, destinée en particulier pour le soin et/ou le maquillage et/ou la protection solaire de la peau et/ou des muqueuses ainsi que pour la préparation d'une crème destinée au traitement des maladies de la peau et/ou des muqueuses.

Dans le domaine cosmétique, il est courant d'utiliser des crèmes constituées d'une émulsion eau-dans-huile (E/H) comportant une phase aqueuse dispersée dans une phase huileuse. Ces émulsions comportent une phase continue huileuse et permettent donc de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches.

Ces émulsions présentent fréquemment des problèmes de stabilité, rendant leur fabrication difficile. Aussi, différents moyens ont été envisagés pour remédier à cet inconvénient. Un moyen consiste à incorporer dans la phase huileuse de l'émulsion, des corps gras solides à la température ambiante tels que les cires ou composés cireux, les silices ou les argiles modifiées, dans le but d'épaissir la phase continue. Toutefois, il en résulté l'obtention de crèmes souvent compactes et lourdes.

Un autre moyen de remédier à l'instabilité des émulsions E/H est l'incorporation d'un électrolyte inorganique tel que le sulfate de magnésium, qui stabilise l'émulsion par effet de répulsion électrostatique évitant les phénomènes de coalescence des globules d'eau dispersés. L'absence d'électrolyte augmente les risques d'instabilité de l'émulsion se traduisant souvent par un déphasage des deux phases. Toutefois, la présence d'électrolyte peut avoir des inconvénients car il arrive que certains composés que l'on souhaite utiliser dans ces émulsions soient incompatibles avec les électrolytes. On sait par exemple que les actifs anioniques forment en présence d'électrolytes inorganiques des sels insolubles et finissent par précipiter plus ou moins rapidement au sein de l'émulsion. Comme actifs anioniques de ce type, on peut citer par exemple les filtres UV hydrosolubles tels que l'acide téréphtalylidène-di-camphosulfonique ou la benzophénone-4.

En outre, il est possible de remédier à l'instabilité des émulsions E/H en augmentant fortement la teneur en émulsionnant de ces émulsions. Or, on sait que les émulsionnants utilisés en grande quantité peuvent se montrer irritants vis-à-vis de certains types de peau. Par ailleurs, comme précédemment, les crèmes obtenues sont souvent compactes et lourdes.

Il est connu par le document RD 95378007 (octobre 1995), ayant trait aux émulsions eau-dans-huile, d'utiliser des galactomannans éthylés pour épaissir des huiles.

Il subsiste donc le besoin d'une émulsion eau-dans-huile ne présentant pas les inconvénients rencontrés avec celles connues jusqu'à présent, cette émulsion étant stable même en présence d'actif sensible aux électrolytes et/ou en absence d'électrolyte et/ou de corps gras solide.

L'émulsion selon l'invention permet notamment de pallier les problèmes mentionnés précédemment. En effet, la demanderesse a trouvé de manière surprenante qu'il était possible d'obtenir une émulsion eau-dans-huile ayant de bonnes propriétés cosmétiques et une bonne stabilité en utilisant un gélifiant particulier.

En particulier, cette émulsion est stable même si elle contient au moins un actif sensible aux électrolytes et/ou qu'elle est exempte d'électrolyte et/ou de corps gras solide.

L'invention a donc pour objet l'utilisation d'un alkyléther de polysaccharide formé de motifs comportant au moins deux cycles osidiques différents, chaque motif comportant au moins un groupe hydroxyle substitué par une chaîne alkyle hydrocarbonée saturée, pour stabiliser une émulsion eau-dans-huile fluide exempte d'électrolyte.

Selon le pourcentage de gélifiant utilisé, on peut obtenir une émulsion de texture plus ou moins fluide. On entend par émulsion de texture fluide, une émulsion s'écoulant sous son propre poids et ayant une viscosité d'environ 2 à 15 poises, soit de 0,2 à 1,5 Pa.s. Une émulsion de texture épaisse selon l'art antérieur a une viscosité d'environ 20 à 80 poises, soit de 2 à 8 Pa.s. L'émulsion selon l'invention présente l'avantage de pouvoir être fluide, crémeuse et confortable tout en présentant une bonne stabilité.

Selon un mode de réalisation particulier de l'invention, l'alkyléther de polysaccharide a un poids moléculaire supérieur à 100 000, et de préférence supérieur à 200 000. Chaque motif peut comporter de un à six et de préférence de deux à quatre groupes hydroxyle substitués par une chaîne alkyle hydrocarbonée saturée.

Par chaîne alkyle hydrocarbonée saturée, on entend une chaîne comportant de 1 à 24, de préférence de 1 à 10 et mieux de 1 à 5 atomes de carbone. En particulier, la chaîne alkyle est choisie parmi les chaînes méthyle, éthyle, n-propyle, isopropyle, n-butyle, isobutyle, tertio-butyle, n-pentyle.

Les cycles osidiques sont notamment choisis parmi le mannose, le galactose, le glucose, le furanose, le rhamnose, l'arabinose.

Selon un mode préféré de réalisation de l'invention, l'alkyléther de polysaccharide selon l'invention est un alkyléther d'une gomme et plus particulièrement d'une gomme globalement non ionique, c'est-à-dire pratiquement sans groupe ionique. Comme gommes appropriées, on peut citer par exemple la gomme guar dont le motif comprend un galactose et un mannose, la gomme de caroube dont le motif comprend un galactose et un mannose, la gomme de karaya qui est un mélange complexe de rhamnose, galactose et acide galacturonique, la gomme adragante qui est un mélange complexe d'arabinose, galactose et acide galacturonique, et leurs mélanges.

Selon un mode de réalisation préféré de l'invention, l'alkyléther de polysaccharide est un dérivé de gomme guar, et plus particulièrement la guar éthylée ayant un degré de substitution d'environ 2 à 3, en particulier 2,5, telle que décrite dans le document RD 95378007 (octobre 1995).

L'émulsion selon l'invention peut contenir par exemple une quantité d'alkyléther de polysaccharide allant de 0,1 à 10 %, et de préférence de 0,5 à 5 % du poids total de l'émulsion.

La quantité d'huile que l'on peut introduire dans l'émulsion peut représenter de 20 % à 80 % du poids total de l'émulsion.

Comme huiles utilisables dans l'invention, on peut citer par exemple les huiles d'origine végétale, les huiles d'origine animale, les huiles de synthèse et notamment les esters gras, et les mélanges de ces huiles, ainsi que les mélanges de ces huiles avec les huiles siliconées, les huiles fluorées et/ou les huiles minérales.

De façon connue, l'émulsion selon l'invention contient un émulsionnant d'émulsion E/H. Comme émulsionnants utilisables dans l'invention, on peut citer par exemple les esters d'acide gras et de glucose tels que le dioléate de méthylglucose, les esters d'acide gras et de glycérine tels que l'isostéarate de glycéryle, l'oléate de glycéryle et le ricinoléate de glycéryle, les esters d'acide gras et de sorbitol tels que le tristéarate de sorbitan et le di- ou tri-oléate de sorbitan, et plus généralement tout émulsionnant ayant un HLB (balance hydrophile-lipophile) inférieur à 6. La quantité d'émulsionnant peut représenter de 0,1 à 20 % et de préférence de 0,2 à 3 % du poids total de l'émulsion.

De préférence, l'émulsion selon l'invention est destinée à un soin ou un traitement topique. Dans ce cas, l'émulsion doit contenir un milieu topiquement acceptable, c'est-à-dire compatible avec la peau, les muqueuses, les ongles, le cuir chevelu et les cheveux. Elle trouve son application dans un grand nombre de traitements cosmétiques et/ou dermatologiques de la peau, y compris du cuir chevelu, en particulier pour le soin et/ou le maquillage et/ou la protection solaire de la peau et/ou des muqueuses, ainsi que pour la préparation d'une crème destinée au traitement des maladies de la peau (peau sèche).

Aussi, la présente invention a encore pour objet l'utilisation de l'émulsion telle que définie ci-dessus dans et/ou pour la fabrication d'une composition cosmétique, pharmaceutique et/ou dermatologique pour traiter la peau et/ou les muqueuses.

La présente invention a aussi pour objet un procédé de traitement cosmétique et/ou dermatologique de la peau sèche, caractérisé par le fait que l'on applique sur la peau sèche une émulsion telle que définie ci-dessus.

De façon connue, l'émulsion de l'invention peut contenir également des adjuvants habituels dans les domaines cosmétique et/ou dermatologique, tels que les actifs, notamment les actifs cosmétiques et/ou dermatologiques pour le soin et/ou le maquillage et/ou la protection solaire de la peau et/ou des muqueuses, les conservateurs, les antioxydants, les agents complexants, les solvants, les parfums, les charges, les filtres, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Les exemples ci-après d'émulsions selon l'invention sont des exemples hypothétiques réalisables donnés à titre d'illustration et sans caractère limitatif. Les quantités y sont données en % en poids.

### Exemple 1:

### Phase huileuse:

- Oléate de glycéryle 4 %
- Huile de vaseline 25 %
- Guar éthylée ayant un degré de substitution d'environ 2,5 1,5 %

### Phase aqueuse :

- Benzophénone-4 (Uvinul MS40) 3 %
- Triéthanolamine 0,8 %
- Eau qsp 100 %

L'émulsion obtenue devrait être crémeuse et stable, sans sulfate de magnésium.

On pourrait l'utiliser notamment pour régler la peau des UVA.

### Contre-exemple 1:

### Phase huileuse :

- Oléate de glycéryle 4 %
- Huile de vaseline 25 %

### Phase aqueuse :

- Benzophénone-4 (Uvinul MS40) 3 %
- Triéthanolamine 0,8 %
- Eau qsp 100 %

L'émulsion du contre-exemple 1 se différencie de l'émulsion de l'exemple 1 par le fait qu'elle ne contient pas de gélifiant selon l'invention. L'émulsion n'est pas stable aux cycles de températures et à la chaleur (étuve à 45°C).

### Contre-exemple 2:

### Phase huileuse :

- Oléate de glycéryle 4 %
- Huile de vaseline 25 %

### Phase aqueuse :

- Benzophénone-4 (Uvinul MS40) 3 %
- Sulfate de magnésium 1,5 %
- Triéthanolamine 0,8 %
- Eau qsp 100 %

L'émulsion du contre-exemple 2 se différencie de l'exemple 1 par le fait que le gélifiant selon l'invention a été remplacé par un électrolyte : le sulfate de magnésium.

L'émulsion est stable mais l'Uvinul MS40 précipite au sein de l'émulsion après quelques jours de conservation à température ambiante.

## Revendications

1. Utilisation d'un alkyléther de polysaccharide formé de motifs comportant au moins deux cycles osidiques différents, chaque motif comportant au moins un groupe hydroxyle substitué par une chaîne alkyle hydrocarbonée saturée, pour stabiliser une émulsion eau-dans-huile fluide exempte d'électrolyte inorganique.

2. Utilisation selon la revendication 1, caractérisée en ce que l'émulsion contient au moins un actif sensible aux électrolytes.

3. Utilisation selon l'une quelconque des revendications précédentes, caractérisée en ce que chaque motif comporte deux à quatre groupes hydroxyle substitués par une chaîne alkyle hydrocarbonée saturée.

4. Utilisation selon l'une quelconque des revendications précédentes, caractérisée en ce que la chaîne alkyle hydrocarbonée saturée comporte de 1 à 24 atomes de carbone.

5. Utilisation selon l'une quelconque des revendications précédentes, caractérisée en ce que la chaîne alkyle hydrocarbonée saturée comporte de 1 à 5 atomes de carbone.

6. Utilisation selon l'une des revendications précédentes, caractérisée en ce que la chaîne alkyle est choisie parmi les chaînes méthyle, éthyle, n-propyle, isopropyle, n-butyle, isobutyle, tertiobutyle, n-pentyle.

7. Utilisation selon l'une quelconque des revendications précédentes, caractérisée en ce que les cycles osidiques sont choisis parmi le mannose, le galactose, le glucose, le furanose, le rhamnose, l'arabinose.

8. Utilisation selon l'une quelconque des revendications précédentes, caractérisée en ce que l'alkyléther de polysaccharide est un alkyléther d'une gomme choisie parmi la gomme guar, la gomme de caroube, la gomme de karaya, la gomme adragante et leurs mélanges.

9. Utilisation selon l'une quelconque des revendications précédentes, caractérisée en ce que l'alkyléther de polysaccharide a un poids moléculaire supérieur à 200 000.

10. Utilisation selon l'une quelconque des revendications précédentes, caractérisée en ce que l'alkyléther de polysaccharide est présent en une quantité allant de 0,1 à 10 % du poids total de la composition.

11. Utilisation selon l'une quelconque des revendications précédentes, caractérisée en ce que l'alkyléther de polysaccharide est présent en une quantité allant de 0,5 à 5 % du poids total de la composition.

12. Utilisation selon l'une quelconque des revendications précédentes, caractérisée en ce que l'émulsion consiste en une composition cosmétique et/ou dermatologique.

13. Utilisation selon l'une quelconque des revendications précédentes, caractérisée en ce que l'émulsion contient au moins un actif cosmétique et/ou dermatologique pour le soin et/ou le maquillage et/ou la protection solaire de la peau et/ou des muqueuses.

## Patentansprüche

1. Verwendung eines Polysaccharidalkylethers, der aus Einheiten gebildet ist, die mindestens zwei verschiedene Zuckerringe aufweisen, wobei jede Einheit mindestens eine Hydroxygruppe enthält, die mit einer gesättigten Alkylgruppe (Kohlenwasserstoffgruppe) substituiert ist, um eine fluide Wasser-in-Öl-Emulsion zu stabilisieren, die keinen anorganischen Elektrolyten enthält.

2. Verwendung nach Anspruch 1, dadurch gekennzeichnet, daß die Emulsion mindestens einen gegenüber Elektrolyten empfindlichen Wirkstoff enthält.

3. Verwendung nach einem der vorhergehenden Ansprüche, dadurch gekennzeichnet, daß jede Einheit 2 bis 4 Hydroxygruppen aufweist, die mit einer gesättigten Alkylgruppe substituiert sind.

4. Verwendung nach einem der vorhergehenden Ansprüche, dadurch gekennzeichnet, daß jede gesättigte Alkylgruppe 1 bis 24 Kohlenstoffatome aufweist.

5. Verwendung nach einem der vorhergehenden Ansprüche, dadurch gekennzeichnet, daß die gesättigte Alkylgruppe 1 bis 5 Kohlenstoffatome aufweist.

6. Verwendung nach einem der vorhergehenden Ansprüche, dadurch gekennzeichnet, daß die Alkylgruppe unter Methyl, Ethyl, n-Propyl, Isopropyl, n-Butyl, Isobutyl, tert.-Butyl und n-Pentyl ausgewählt ist.

7. Verwendung nach einem der vorhergehenden Ansprüche, dadurch gekennzeichnet, daß die Zuckerringe sind insbesondere unter Mannose, Galactose, Glucose, Furanose, Rhamnose und Arabinose ausgewählt sind.

8. Verwendung nach einem der vorhergehenden Ansprüche, dadurch gekennzeichnet, daß der Polysaccharidalkylether ein Alkylether eines Gummis ist, das unter Guarmehl, Johannisbrotkernmehl, Karayagummi und Traganth und deren Gemischen ausgewählt ist.

9. Verwendung nach einem der vorhergehenden Ansprüche, dadurch gekennzeichnet, daß der Polysaccharidalkylether ein Molekulargewicht über 200 000 aufweist.

10. Verwendung nach einem der vorhergehenden Ansprüche, dadurch gekennzeichnet, daß der Polysaccharidalkylether in einem Mengenanteil im Bereich von 0,1 bis 10 des Gesamtgewichts der Zusammensetzung vorliegt.

11. Verwendung nach einem der vorhergehenden Ansprüche, dadurch gekennzeichnet, daß der Polysaccharidalkylether in einem Mengenanteil im Bereich von 0,5 bis 5 % des Gesamtgewichts der Zusammensetzung vorliegt.

12. Verwendung nach einem der vorhergehenden Ansprüche, dadurch gekennzeichnet, daß die Emulsion aus einer kosmetischen und/oder dermatologischen Zusammensetzung besteht.

13. Verwendung nach einem der vorhergehenden Ansprüche, dadurch gekennzeichnet, daß die Emulsion mindestens einen kosmetischen und/oder dermatologischen Wirkstoff zur Pflege und/oder zum Schminken und/oder zum Sonnenschutz der Haut und/oder der Schleimhäute enthält.

## Claims

1. Use of a polysaccharide alkyl ether formed from units containing at least two different monosaccharide rings, each unit containing at least one hydroxyl group substituted by a saturated hydrocarbon alkyl chain, for stabilizing a fluid water-in-oil emulsion which is devoid of inorganic electrolyte.

2. Use according to Claim 1, characterized in that the emulsion contains at least one active principle sensitive to electrolytes.

3. Use according to any one of the preceding claims, characterized in that each unit contains two to four hydroxyl groups substituted by a saturated hydrocarbon alkyl chain.

4. Use according to any one of the preceding claims, characterized in that the saturated hydrocarbon alkyl chain contains from 1 to 24 carbon atoms.

5. Use according to any one of the preceding claims, characterized in that the saturated hydrocarbon alkyl chain contains from 1 to 5 carbon atoms.

6. Use according to one of the preceding claims, characterized in that the alkyl chain is chosen from methyl, ethyl, n-propyl, isopropyl, n-butyl, isobutyl, tert-butyl and n-pentyl chains.

7. Use according to any one of the preceding claims, characterized in that the monosaccharide rings are chosen from mannose, galactose, glucose, furanose, rhamnose and arabinose.

8. Use according to any one of the preceding claims, characterized in that the polysaccharide alkyl ether is an alkyl ether of a gum chosen from guar gum, locust bean gum, karaya gum, gum tragacanth and their mixtures.

9. Use according to any one of the preceding claims, characterized in that the polysaccharide alkyl ether has a molecular weight of greater than 200,000.

10. Use according to any one of the preceding claims, characterized in that the polysaccharide alkyl ether is present in an amount ranging from 0.1 to 10% of the total weight of the composition.

11. Use according to any one of the preceding claims, characterized in that the polysaccharide alkyl ether is present in an amount ranging from 0.5 to 5% of the total weight of the composition.

12. Use according to any one of the preceding claims, characterized in that the emulsion is composed of a cosmetic and/or dermatological composition.

13. Use according to any one of the preceding claims, characterized in that the emulsion contains at least one cosmetic and/or dermatological active principle for caring for and/or making up the skin and/or mucous membranes and/or for the antisun protection of the skin and/or mucous membranes.
